Europäisches Patentamt

European Patent Office

Office européen des brevets

(11) Veröffentlichungsnummer: **0 353 459**

**A1**

(12) **EUROPÄISCHE PATENTANMELDUNG**

(21) Anmeldenummer: **89111790.5**

(51) Int. Cl.⁴: **A61B 6/00**

(22) Anmeldetag: **28.06.89**

(30) Priorität: **13.07.88 US 218378**

(43) Veröffentlichungstag der Anmeldung:
**07.02.90 Patentblatt 90/06**

(84) Benannte Vertragsstaaten:
**BE DE FR GB**

(71) Anmelder: **Siemens Aktiengesellschaft**
**Wittelsbacherplatz 2**
**D-8000 München 2(DE)**

(72) Erfinder: **Faul, David-D.**
**21 Canal Street**
**Yardley, PA 19067(US)**
Erfinder: **Greiner, Hans-Joachim, Dipl.-Ing.**
**Am Eichengarten 1**
**D-8520 Erlangen(DE)**
Erfinder: **Wessels, Gerd, Dr.**
**Tetzelweg 42**
**D-8520 Erlangen(DE)**

(54) **Röntgentechnische Anlage.**

(57) Bei einer Röntgendiagnostikanlage soll eine berührungslose Dateneingabe erfolgen, wobei Mittel zur Kommunikation des Benutzers mit Komponenten der Anlage vorhanden sind.

Die Komponenten (1, 2, 4, 6) der Anlage sind über eine Ringleitung (3) zusammengeschaltet, in der ein Spracherkennungssystem (6) zur Dateneingabe und eine Anzeigevorrichtung (8) liegen, die derart ausgebildet ist, daß auf ihr die verfügbaren und ausgeführten Kommandos wiedergegeben werden.

# Röntgentechnische Anlage

Die Erfindung betrifft eine röntgentechnische Anlage mit einem Röntgengenerator, einem Röntgengerät, einem Eingabesystem für Daten und einer Anzeigevorrichtung für die Datenwiedergabe.

Bei bekannten Röntgendiagnostikanlagen erfolgt die Dateneingabe, z.B. die Eingabe von Aufnahmewerten, mit Hilfe mechanisch betätigbarer Schaltmittel, z.B. mit Hilfe von Tasten oder Drehschaltern. Die Bedienungsperson muß beim Arbeiten mit einer Röntgendiagnostikanlage die Hände auch für andere Tätigkeiten, z.B. medizinische Manipulationen, frei haben. Die Dateneinstellung mit Hilfe von manuell betätigbaren Schaltmitteln ist demgemäß störend.

Es ist bereits bekannt, einen Dentalarbeitsplatz oder verstellbare Komponenten einer Röntgenanlage zur Behandlung von Patienten durch Sprachbefehle zu steuern. Die Bedienungsperson braucht in diesem Fall ihre Hände zur Auslösung von Steuervorgängen nicht zu benutzen, was im Hinblick darauf sehr günstig ist, daß für viele medizinische Manipulationen Sterilität erforderlich ist.

Der Erfindung liegt die Aufgabe zugrunde, eine röntgentechnische Anlage der eingangs genannten Art so auszubilden, daß eine einfache Kommunikation zwischen dem Benutzer und allen Kommponenten der Anlage möglich ist.

Diese Aufgabe ist erfindungsgemäß dadurch gelöst, daß die Komponenten der Anlage über eine Ringleitung zusammengeschaltet sind, in der ein Spracherkennungssystem zur Dateneingabe und die Anzeigevorrichtung liegen, die derart gesteuert ist, daß auf ihr die verfügbaren und ausgeführten Kommandos wiedergegeben werden. Bei der erfindungsgemäßen Anlage ist über die anzeigevorrichtung, die vorzugsweise ein von einer Fernsehzentrale gesteuerter Monitor sein kann, eine Kommunikation mit dem Röntgengenerator und dem Röntgengerät in der Weise möglich, daß die Bedienungsperson auf der Anzeigevorrichtung feststellt, welche Kommandos verfügbar sind, die jeweils gewünschten Kommandos nacheinander eingibt und die ausgeführten Kommandos auf der Anzeigevorrichtung kontrolliert.

Eine zusätzliche Information der Bedienungsperson über die verfügbaren Kommandos kann erfolgen, wenn eine Lautsprecheranlage zur akustischen Wiedergabe dieser Kommandos vorhanden ist. Zusätzlich zum Spracherkennungssystem können auch mechanisch betätigbare Schaltmittel zur Dateneingabe vorhanden sein, die es ermöglichen, kontinuierliche Steuerungen, z.B. kontinuierliche Verstellvorgänge, durchzuführen. Die Lautsprecheranlage kann die Bedienungsperson auch auf Fehleingaben aufmerksam machen und die ausgeführten Befehle wiederholen.

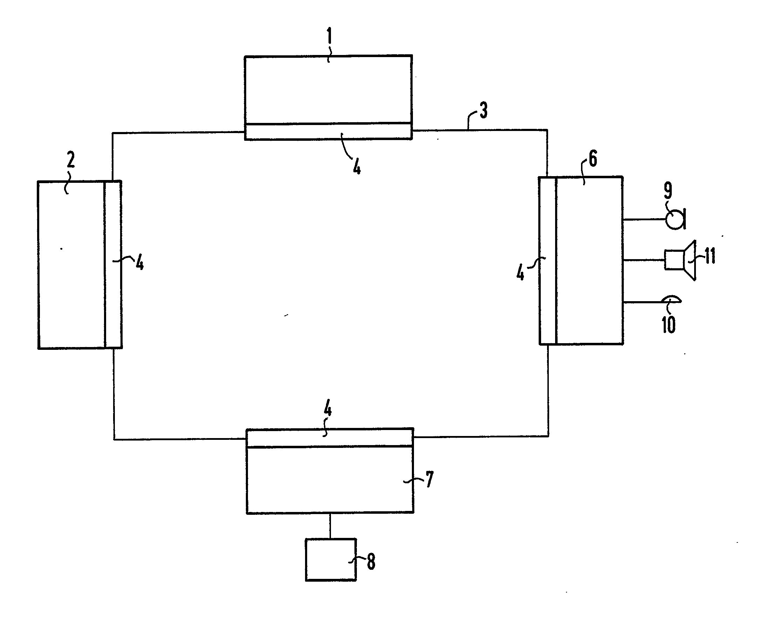

Die Erfindung ist nachfolgend anhand eines in der Zeichnung dargestellten Ausführungsbeispieles näher erläutert.

In der Zeichnung ist eine Röntgenanlage mit einem Röntgengenerator 1 und einem Röntgengerät 2 dargestellt, die durch eine Ringleitung 3 miteinander verbunden sind. Die Ankopplung des Röntgengenerators 1 und des Röntgengerätes 2 an die Ringleitung 3 erfolgt durch Röntgenanlagenschnittstellen 4. In der Ringleitung liegen ein Spracherkennungssystem 6 und eine Fernsehzentrale 7, die ebenfalls über Röntgenanlagenschnittstellen 4 an der Ringleitung 3 angekoppelt sind. An der Fernsehzentrale 7 ist ein Monitor 8 angeschlossen.

Dem Spracherkennungssystem 6 ist ein Mikrophon 9 zugeordnet, über das die Bedienungsperson der Anlage Befehle und/oder Daten in Form von Sprachkommandos eingeben kann. Ein Fußschalter 10 dient zur Realisierung kontinuierlicher Steuervorgänge. Das Spracherkennungssystem 6 setzt die eingegebenen Sprachinformationen in maschinengerechte Steuerbefehle und/oder Daten um, die über die Röntgenanlagenschnittstelle 4 auf die Ringleitung 3 gegeben werden und den Röntgengenerator 1 und/oder das Röntgengerät 2 über die Röntgenanlagenschnittstellen 4 steuern. Das Spracherkennungssystem 6 ist zusätzlich an einer Lautsprecheranlage 11 angeschlossen. Die Lautsprecheranlage 11 macht den Benutzer auf Fehlermeldungen aufmerksam und wiederholt die ausgeführten Befehle.

Die Fernsehzentrale 7, die den Monitor 8 steuert, enthält Informationen über die verfügbaren Kommandos zur sprachgesteuerten Befehlsund/oder Dateneingabe. Die verfügbaren Kommandos werden der Bedienungsperson auf dem Monitor 8 vorzugsweise im Klartext angezeigt. Ferner enthält die Fernsehzentrale 7 über die Röntgenanlagenschnittstelle 4 Informationen über die jeweils ausgeführten, über die Sprache eingegebenen Kommandos und/oder Daten und zeigt auch diese Kommandos und/oder Daten auf dem Monitor 8 der Bedienungsperson an. Durch Beobachtung des Monitors 8 kann die Bedienungsperson demgemäß erkennen, welche Kommandos sie eingeben kann und/oder welche Kommandos bereits ausgeführt worden sind.

## Ansprüche

1. Röntgentechnische Anlage mit einem Röntgengenerator (1), einem Röntgengerät (2), einem Eingabesystem (6, 9, 10) für Daten und einer Anzeigevorrichtung (7, 8) für die Datenwiedergabe, **dadurch gekennzeichnet,** daß die Komponenten (1, 2, 6, 7) der Röntgenanlage über eine Ringleitung (3) zusammengeschaltet sind, in der ein Spracherkennungssystem (6) zur Dateneingabe und die Anzeigevorrichtung (7, 8) liegt, die derart gesteuert ist, daß auf ihr die verfügbaren und ausgeführten Kommandos wiedergegeben werden.

2. Anlage nach Anspruch 1, **dadurch gekennzeichnet,** daß eine Lautsprecheranlage (11) zur akustischen Wiedergabe der Kommandos vorhanden ist.

3. Anlage nach Anspruch 1 oder 2, **dadurch gekennzeichnet,** daß die Anzeigevorrichtung von einem von einer Fernsehzentrale (7) gesteuerten Monitor (8) gebildet ist.

4. Anlage nach einem der Ansprüche 1 bis 3, **dadurch gekennzeichnet,** daß zusätzlich zum Spracherkennungssystem (6) mechanisch betätigbare Schaltmittel (10) zur Dateneingabe vorhanden sind.

Europäisches Patentamt

**EUROPÄISCHER RECHERCHENBERICHT**

Nummer der Anmeldung

EP 89 11 1790

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | FR-A-2 594 321 (J.-M. INGLESE et al.) * Zusammenfassung; Seite 1, Zeilen 21-30; Seite 2, Zeilen 6-26; Seite 3, Zeile 12 - Seite 4, Zeile 11, Seite 4, Zeile 20 - Seite 5, Zeile 15; Seiten 6,7; Ansprüche 1-8,13,14; Figur 1/1 * --- | 1-4 | A 61 B 6/00 |
| Y | EP-A-0 245 160 (THOMSON-CGR) * Zusammenfassung; Seite 2, Zeilen 5-14; Seite 4, Zeile 20 - Seite 6, Zeile 24; Seite 7, Zeile 7 - Seite 8, Zeile 15; Figuren 1-4 * --- | 1,3 | |
| A | DE-A-3 636 678 (SIEMENS AG) * Zusammenfassung; Spalte 1, Zeilen 1-19,30-44; Figuren 1-3 * ----- | 1-3 | |

|  | RECHERCHIERTE SACHGEBIETE (Int. Cl.5) |
|---|---|
|  | A 61 B H 04 L H 05 G |

Der vorliegende Recherchenbericht wurde für alle Patentansprüche erstellt

| Recherchenort | Abschlußdatum der Recherche | Prüfer |
|---|---|---|
| DEN HAAG | 16-10-1989 | RIEB K.D. |